(19) **Europäisches Patentamt / European Patent Office / Office européen des brevets**

(11) **EP 4 763 222 A1**

(12) **EUROPEAN PATENT APPLICATION**

(43) Date of publication:
24.06.2026 Bulletin 2026/26

(21) Application number: 25211886.4

(22) Date of filing: 29.10.2025

(51) International Patent Classification (IPC):
*A61K 39/395* (2006.01)    *C07K 16/22* (2006.01)

(52) Cooperative Patent Classification (CPC):
**C07K 16/22; A61K 39/39591;** C07K 2317/40;
G01N 33/6848

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: 25.08.2025 CN 202511190218

(71) Applicant: **QILU PHARMACEUTICAL CO., LTD.**
**Jinan, Shandong 250100 (CN)**

(72) Inventors:
- **AN, Zhenming**
  **Jinan, Shandong, 250100 (CN)**
- **LIU, Dandan**
  **Jinan, Shandong, 250100 (CN)**
- **ZHANG, Xiaoyun**
  **Jinan, Shandong, 250100 (CN)**
- **LI, Tao**
  **Jinan, Shandong, 250100 (CN)**
- **YU, Yue**
  **Jinan, Shandong, 250100 (CN)**
- **LI, Qiyu**
  **Jinan, Shandong, 250100 (CN)**
- **LI, Daoyuan**
  **Jinan, Shandong, 250100 (CN)**
- **LIU, Chuanlei**
  **Jinan, Shandong, 250100 (CN)**
- **YE, Hongyan**
  **Jinan, Shandong, 250100 (CN)**

(74) Representative: **Bandpay & Greuter**
**11, rue Christophe Colomb**
**75008 Paris (FR)**

Remarks:
The complete document including Reference Table(s) and the Sequence Listing(s) can be downloaded from the EPO website

(54) **A COMPOSITION COMPRISING APITEGROMAB**

(57) The present application provides a composition comprising Apitegromab and its variants, wherein the variants comprise mono-oxidized or di-oxidized variants resulting from oxidation at individual sites of Apitegromab. Also provided are a pharmaceutical formulation comprising the composition, and related use thereof. Further, provided is a method for detecting the variants.

**Description**

**Cross-Reference to Related Application**

The present application claims priority to Chinese Patent Application No. 202511190218.2, filed on August 25, 2025, the entire contents of which are incorporated herein by reference for all purposes.

**Technical Field**

The present application relates to the field of pharmaceutical formulations. In particular, the present application provides a composition comprising Apitegromab and variants thereof, as well as related methods and uses.

**Background of the Invention**

Myostatin, also known as GDF-8, is a key factor regulating muscle growth, and can maintain the balance of muscle mass by inhibiting the proliferation and differentiation of muscle cells. Abnormal function or excessive expression of Myostatin can lead to muscle atrophy diseases, such as spinal muscular atrophy (SMA) and other neuromuscular disorders.

Apitegromab is a Myostatin inhibitor, and is a fully humanized monoclonal antibody of the IgG4 subtype. By selectively inhibiting activation of Myostatin, Apitegromab can promote muscle growth and improve motor function, providing a novel muscle-targeted treatment strategy for patients with SMA.

As a medicine, Apitegromab must maintain its stability and efficacy. The quality control of a pharmaceutical composition is mainly concerned with the control of the contents of active ingredients and related substances such as variants or impurities. In particular, the contents of the related substances need to meet the medicinal requirements.

**Summary of the Invention**

In a first aspect, the present application provides a composition comprising Apitegromab and a variant thereof, wherein the variant comprises a mono-oxidized variant resulting from oxidation of tryptophan at position 107 in the amino acid sequence of the heavy chain of Apitegromab, and a mono-oxidized variant resulting from oxidation of methionine at position 113 in the amino acid sequence of the heavy chain of Apitegromab, and the positions are numbered with reference to the sequence as set forth in SEQ ID NO:1.

In a second aspect, the present application provides a composition comprising Apitegromab and a variant thereof, wherein the variant comprises a di-oxidized variant resulting from oxidation of tryptophan at position 107 in the amino acid sequence of the heavy chain of Apitegromab, and the position is numbered with reference to the sequence as set forth in SEQ ID NO:1.

In some embodiments, Apitegromab comprises a heavy chain having an amino acid sequence as set forth in SEQ ID NO:1, and a light chain having an amino acid sequence as set forth in SEQ ID NO:2.

In some embodiments, the variant is an oxidized variant resulting from oxidation within the CDR3 of the heavy chain of Apitegromab.

In some embodiments, the variant in the composition disclosed in the first aspect further comprises a di-oxidized variant resulting from oxidation of tryptophan at position 107 in the amino acid sequence of the heavy chain of Apitegromab.

In some embodiments, the variant is a mono-oxidized variant resulting from oxidation of tryptophan at position 107 in the amino acid sequence of the heavy chain of Apitegromab, and a mono-oxidized variant resulting from oxidation of methionine at position 113 in the amino acid sequence of the heavy chain of Apitegromab, wherein the positions are numbered with reference to the sequence as set forth in SEQ ID NO:1.

In some embodiments, the variant is a di-oxidized variant resulting from oxidation of tryptophan at position 107 in the amino acid sequence of the heavy chain of Apitegromab, wherein the position is numbered with reference to the sequence as set forth in SEQ ID NO:1.

In particular embodiments, the sum of the contents of the two mono-oxidized variants is less than or equal to about 40% of the total content of Apitegromab and the variant thereof in the composition, calculated based on a peptide mass fingerprinting analysis.

In particular embodiments, the content of the di-oxidized variant is less than or equal to about 45% of the total content of Apitegromab and the variant thereof in the composition, calculated based on a peptide mass fingerprinting analysis.

In particular embodiments, the variant has essentially similar antigen-binding affinity to Apitegromab.

In a third aspect, the present application provides a pharmaceutical formulation, comprising the composition disclosed in the first or second aspect, and one or more pharmaceutically acceptable carriers

In a fourth aspect, the present application provides a method for preparing the pharmaceutical formulation in the third aspect, comprising the steps of:

1) preparing a composition comprising Apitegromab and variants thereof, wherein the variants are the oxidized variants as defined in the first aspect; and
2) evaluating the mono-oxidized variant or the di-oxidized variant in the composition; and optionally confirming that the sum of the contents of the two mono-oxidized variants is less than or equal to about 40%, and/or confirming that the content of the di-oxidized variant is less than or equal to about 45%.

In some embodiments, the step of 2) comprises obtaining XIC chromatograms of the mono-oxidized peptide segment, the di-oxidized peptide segment and the native peptide segment by performing peptide mass fingerprinting analysis on the composition, and calculating the content of the mono-oxidized variant or the di-oxidized variant from the corresponding peak areas.

In some embodiments, the method further comprises combining the composition with a pharmaceutically acceptable carriers after the step of 2).

In a fifth aspect, the present application provides a method for detecting an Apitegromab variant in a composition or pharmaceutical formulation comprising Apitegromab, wherein the variant is i) a mono-oxidized variant resulting from oxidation of tryptophan at position 107 in the amino acid sequence of the heavy chain of Apitegromab, and a mono-oxidized variant resulting from oxidation of methionine at position 113 in the amino acid sequence of the heavy chain of Apitegromab; or ii) a di-oxidized variant resulting from oxidation of tryptophan at position 107 in the amino acid sequence of the heavy chain of Apitegromab, and wherein the positions are numbered with reference to the sequence as set forth in SEQ ID NO:1; and the method comprises:

1) subjecting the composition or pharmaceutical formulation to trypsin digestion to obtain the digestion product;
2) subjecting the digestion product to a peptide mass fingerprinting analysis (e.g., by using liquid chromatography-tandem mass spectrometry, LC-MS/MS); and determining the presence or absence of the variant based on MS1 and MS2 spectra.

In some embodiments, the method further comprises obtaining XIC chromatograms of the mono-oxidized peptide segment, the di-oxidized peptide segment and the native peptide segment after performing peptide mass fingerprinting analysis when the variant is determined to be present after performing the peptide mass fingerprinting analysis; and calculating the contents of the oxidized variants from the corresponding peak areas.

In a sixth aspect, the present application provides use of the composition disclosed in the first aspect or the second aspect, or the pharmaceutical formulation disclosed in the third aspect in the preparation of a medicament for the treatment of a neuromuscular disease.

In some embodiments, the neuromuscular disease is a muscular atrophy disease, preferably spinal muscular atrophy, sarcopenia or Duchenne muscular dystrophy.

In a seventh aspect, the present application provides a method for treating a neuromuscular disease, comprising administering a therapeutically effective dose of the composition disclosed in the first aspect or the second aspect, or the pharmaceutical formulation disclosed in the third aspect to a subject or a patient in need thereof. In some embodiments, the neuromuscular disease is a muscular atrophy disease, preferably spinal muscular atrophy, sarcopenia or Duchenne muscular dystrophy.

## Brief Description of the Drawings

FIG. 1 shows the structures of the mono-oxidized or di-oxidized variants of Apitegromab.
FIG. 2 shows a schematic diagram of the structures of tryptophan-oxidized and methionine-oxidized variants.
FIG. 3 shows the extracted ion chromatogram of MS1 of the mono-oxidized variants (A), MS1 spectrum of the mono-oxidized variants (B), MS2 spectrum of the HC:W$^{107}$ mono-oxidized variant (C), and MS2 spectrum of the HC:W$^{113}$ mono-oxidized variant (D) in an Apitegromab sample.
FIG. 4 shows the extracted ion chromatogram of MS1 (A), MS1 spectrum (B), and MS2 spectrum (C) of the HC:W$^{107}$ di-oxidized variant in an Apitegromab sample.
FIG. 5 shows the extracted ion chromatograms of the mono-oxidized variant peptide segment, the di-oxidized variant peptide segment, and the native peptide segment in an Apitegromab sample.
FIG. 6 shows sensorgrams of affinity analysis of Apitegromab samples for recombinant human Myostatin.

**Description of the Sequences**

SEQ ID NO: 1 is the amino acid sequence of the two identical heavy chains of Apitegromab shown as below (Note: the underlined portions represent the CDR domains, annotated according to the Kabat numbering scheme):

QVQLVESGGGVVQPGRSLRLSCAASGFTFS<u>SYGMH</u>WVRQAPGKGLEWVA<u>VIS
YDGSNKYYADSVKG</u>RFTISRDNSKNTLYLQMNSLRAEDTAVYYCAR<u>DLLVRF
LEWSHYYGMDV</u>WGQGTTVTVSSASTKGPSVFPLAPCSRSTSESTAALGCLVK
DYFPEPVTVSWNSGALTSGVHTFPAVLQSSGLYSLSSVVTVPSSSLGTKTYTCN
VDHKPSNTKVDKRVESKYGPPCPPCPAPEFLGGPSVFLFPPKPKDTLMISRTPEV
TCVVVDVSQEDPEVQFNWYVDGVEVHNAKTKPREEQFNSTYRVVSVLTVLH
QDWLNGKEYKCKVSNKGLPSSIEKTISKAKGQPREPQVYTLPPSQEEMTKNQV
SLTCLVKGFYPSDIAVEWESNGQPENNYKTTPPVLDSDGSFFLYSRLTVDKSR
WQEGNVFSCSVMHEALHNHYTQKSLSLSLG (SEQ ID NO: 1)

SEQ ID NO: 2 is the amino acid sequence of the two identical light chains of Apitegromab shown as below (Note: the underlined portions represent the CDR domains, annotated according to the Kabat numbering scheme):

QSVLTQPPSASGTPGQRVTISC<u>SGSSSNIGSNTVH</u>WYQQLPGTAPKLLIY<u>SDNQR
PS</u>GVPDRFSGSKSGTSASLAISGLQSEDEADYYC<u>AAWDDSLNGV</u>FGGGTKLTV
LGQPKAAPSVTLFPPSSEELQANKATLVCLISDFYPGAVTVAWKADSSPVKAG
VETTTPSKQSNNKYAASSYLSLTPEQWKSHRSYSCQVTHEGSTVEKTVAPTEC
S (SEQ ID NO: 2)

**Detailed Description**

Myostatin is a key factor in maintaining the dynamic balance of muscle mass. It maintains the balance of muscle mass by inhibiting the proliferation and differentiation of muscle cells, and its pathological upregulation can lead to muscle atrophy. Abnormally elevated level of Myostatin in a patient suffering from sarcopenia, Duchenne muscular dystrophy and other diseases can lead to reduced muscle mass, decreased muscle strength and decreased exercise capacity. Blocking the activity of Myostatin has become an important strategy for treating diseases related to muscle atrophy.

Apitegromab is a Myostatin inhibitor, and formed by two identical heavy chains and two identical light chains via disulfide bonds. In particular embodiments, the sequece of the two identical heavy chains of Apitegromab is shown in SEQ ID NO:1, and the sequece of the two identical light chains of Apitegromab is shown in SEQ ID NO:2.

The inventors of the present application have surprisingly found that oxidized variants of Apitegromab are generated during the preparation of Apitegromab, which are mono-oxidized or di-oxidized variants resulting from the oxidation within the CDR3 of the heavy chain of Apitegromab. The presence of the variants does not affect the antigen-binding affinity or related activities of an Apitegromab sample. Therefore, during the preparation of Apitegromab, there is no need to carry out targeted removal strategies for these oxidized variants, in particular if the mono-oxidized variant of Apitegromab is in an amount of no higher than 40%, or the di-oxidized variant of Apitegromab is in an amount of no higher than 45%. This will simplify the production process and reduce the production cost to a certain extent.

In some embodiments, the activities of the composition comprising Apitegromab and its variants are detected, including affinity and binding activity to the antigen Myostatin, as well as related biological activities, etc.

In some embodiments, the antigen-binding affinity of Apitegromab and variants thereof in the composition disclosed herein is detected by surface plasmon resonance (SPR) analysis. In particular embodiments, the oxidized variants of Apitegromab has essentially similar antigen-binding affinity or biological activity to Apitegromab.

In particular embodiments, the inventors have identified the above variants and found that the difference between the variants and Apitegromab includes: i) mono-oxidation occurred on tryptophan at position 107 and on methionine at position 113 within the CDR3 of the heavy chain; or ii) di-oxidation occurred on tryptophan at position 107 within the CDR3 of the heavy chain. The variants herein are named "oxidized variants".

In some embodiments, in the composition comprising Apitegromab and a variant thereof disclosed herein, the Apitegromab variant is an oxidized variant, and the oxidized variant has no significant effect on the affinity or binding activity of Apitegromab for Myostatin.

It is well known in the art that for an antibody, the antigen-binding site is composed of CDR1, CDR2 and CDR3 of the heavy chain and CDR1, CDR2 and CDR3 of the light chain, which directly determine the specificity and affinity of an antibody for its antigen. In particular, CDR3 of the heavy chain is the primary contributor to both affinity and specificity. The inventors of the present application have unexpectedly found that oxidized variants resulting from oxidation within the CDR3 of the heavy chain do not weaken the binding activity and affinity of Apitegromab for its antigen, even at high levels of such oxidized variants.

Described herein is the preparation of Apitegromab using cell culture medium. In some embodiments, the cell medium is Trans MaxC1 medium.

In some embodiments, Apitegromab is expressed in suitable host cells. Nonlimiting examples of such host cells include, but are not limited to, ExpiCHOS, CHO, CHO K1, NS0, Sp2/0, embryonic kidney cells, BHK.

In some embodiments, the Apitegromab protein is purified using Protein A magnetic beads, and the final purified protein is analyzed by Size Exclusion Chromatography (SEC) for purity.

In some embodiments, the sample to be tested is subjected to a peptide mass fingerprinting analysis by LC-MS/MS method.

In the present application, during the process of using the LC - MS/MS method to perform peptide mass fingerprinting analysis on the enzymatically digested Aptigumab samples, the inventors have unexpectedly found oxidized variants, including the mono-oxidized variant of tryptophan, the di-oxidized variant of tryptophan, and the mono-oxidized variant of methionine. Among them, the molecular weight of the mono-oxidized variant increased by 16 Da, and the molecular weight of the di-oxidized variant increased by 32 Da. All of these variations occur in the peptide segment within the CDR3 of the heavy chain.

In particular embodiments, during the peptide mass fingerprinting analysis, the CDR3 sequence of the heavy chain of Apitegromab is DLLVRFLEWSHYYGMDV (SEQ ID NO: 3). After trypsin digestion, the peptide segment containing part of the CDR3 sequence is FLEWSHYYGMDVWGQGTTVTVSSASTK (SEQ ID NO: 4), with the underlined positions indicating the modification sites. Here, W and M have monooxidized forms, and W also has a dioxygenated form.

In some embodiments, liquid chromatography-tandem mass spectrometry (LC-MS/MS) analysis is used to detect the mono-oxidized and di-oxidized variants in an Apitegromab product.

In particular embodiments, after the mass spectrometry data are obtained, extracted ion chromatograms (XIC) of two charge forms which have the strongest responses to the modified peptide segment and its corresponding native peptide segment are extracted, and integrated to obtain respective peak areas. The contents of the mono-oxidized and di-oxidized variants are calculated according to the following formulas:

The content of mono-oxidized variant = (XIC area of mono-oxidized peptide segment)/(XIC area of mono-oxidized peptide segment + XIC area of di-oxidized peptide segment + XIC area of native peptide segment) × 100% The content of di-oxidized variant = (XIC area of di-oxidized peptide segment)/(XIC area of mono-oxidized peptide segment + XIC area of di-oxidized peptide segment + XIC area of native peptide segment) × 100%

In particular embodiments, in the composition comprising Apitegromab and a variant thereof disclosed herein, the content of the two mono-oxidized variants (i.e., the mono-oxidized variant resulting from oxidation of tryptophan at position 107, and the mono-oxidized variant resulting from oxidation of methionine at position 113) is about 0.01% to about 40%, about 0.1% to about 40%, about 1% to about 40%, about 2% to about 30%, about 2% to about 35%, about 2% to about 38%, about 2% to about 40%, or any interval within any of the above ranges or any value within the interval, e.g. 2.38%, 32.41%, 38.20%, of the total content of Apitegromab and its variants.

In particular embodiments, in the composition comprising Apitegromab and a variant thereof disclosed herein, the content of the di-oxidized variant (i.e., the di-oxidized variant resulting from di-oxidation of tryptophan at position 107) is about 0.01% to about 45%, about 0.1% to about 45%, about 0.5% to about 45%, about 1% to about 45%, about 0.5% to about 1%, about 1% to about 40%, or any interval within any of the above ranges or any value within the interval, e.g. 0.58%, 0.68%, 41.58%, of the total content of Apitegromab and its variants.

In more particular embodiments, the method for identifying an oxidized variant and calculating proportion thereof disclosed herein is as follows:

A certain amount of Apitopegumab sample was taken and added to a solution with a final concentration of 6M urea and 10mM TCEP, and then incubated at 37°C for 20min for denaturation and reduction. After that, iodoacetamide (IAM) was added to a final concentration of 20mM, and the sample was incubated at 37°C for 15min for alkylation. After completion, the urea concentration is diluted to 0.6 M by adding 50 mM ammonium bicarbonate, and then sequencing grade trypsin is added at a protein-to-enzyme ratio of 10:1 (w/w) for digestion at 37°C for 25 min. The reaction is terminated by adding formic acid at a final concentration of 1 %.

After centrifugation, the sample is subjected to peptide mass fingerprinting analysis by liquid chromatography-tandem high-resolution mass spectrometry. The peptide segments are separated by C18 column and then analyzed by MS1 and MS2. Then XICs of the mono-oxidized modification form (the mass-to-charge ratios of the main ions are 1018.47 and 1527.20, respectively), di-oxidized modification form (the mass-to-charge ratios of the main ions are 1023.80 and 1535.20, respectively), and its corresponding unmodified form (the mass-to-charge ratios of the main ions are 1013.14 and 1519.21, respectively) of the peptide segment FLEWSHYYGMDVWGQGTTVTVSSASTK (SEQ ID NO: 4) are extracted respectively, and integrated to obtain respective peak areas of each target component. The modification proportion is calculated according to the following formulas:

The content of mono-oxidized variant = (XIC area of mono-oxidized peptide segment)/(XIC area of mono-oxidized peptide segment + XIC area of di-oxidized peptide segment + XIC area of native peptide segment) $\times$ 100%

The content of di-oxidized variant = (XIC area of di-oxidized peptide segment)/(XIC area of mono-oxidized peptide segment + XIC area of di-oxidized peptide segment + XIC area of native peptide segment) $\times$ 100%

In particular embodiments, an Apitegromab sample is oxidized by an oxidant to obtain a forced oxidization sample with higher contents of mono-oxidized and di-oxidized variants.

In particular embodiments, treatment of an Apitegromab sample with 2,2'-azobis(2-amidinopropane) dihydrochloride (AAPH) significantly increases the contents of both mono-oxidized and di-oxidized variants. In another embodiment, treatment of an Apitegromab sample with hydrogen peroxide ($H_2O_2$) significantly increases the content of the mono-oxidized variants, but does not significantly change the content of the di-oxidized variant.

Also disclosed herein is the preparation of a pharmaceutical formulation of the composition disclosed herein, i.e., mixing the composition with an optional pharmaceutically acceptable carrier, and storing in a lyophilized formulation or an aqueous solution. The pharmaceutically acceptable carrier is non-toxic to a recipient at the dosages and concentrations employed. In some embodiments, the pharmaceutically acceptable excipient includes, for example, water; buffers such as phosphates, citrates and other organic acids; antioxidant such as ascorbic acid and methionine; preservatives; hydrophilic polymers such as polyvinylpyrrolidone; and chelating agents such as EDTA. In some embodiments, the formulations for *in vivo* administration must be sterilized, which can be easily achieved by filtration with a sterile filtration membrane.

In this specification and in the claims, the term "about" or "substantially" refers to a numerical range considered by one of ordinary skill in the art to be equivalent to the recited numerical value (e.g., having the same function or result), e.g., +/-10% of the recited numerical value.

In this specification and in the claims, the phrases "include", "comprise" and "contain" mean "include but are not limited to", and does not intended to exclude other parts, additives, components or steps.

It should be understood that features, characteristics, components, or steps described in a particular aspect, embodiment, or example of the present application can be applied to any other aspect, embodiment, or example described herein unless contradicted thereby.

The above disclosure generally describes the present invention, which will be further exemplified by the following Examples. These Examples are described merely to illustrate the invention and are not intended to limit the scope of the invention. Although specific terms and values are used herein, such terms and values are also understood to be exemplary and do not limit the scope of the invention. Unless otherwise specified, the experimental methods and techniques in this specification are those well known to a person skilled in the art.

**EXAMPLES**

**Example 1 Preparation of Apitegromab**

Upstream cell culture process

In this Example, ExpiCHO-S cells were used for protein expression with pcDNA3.4 as the expression vector. DNA fragments encoding the antibody heavy and light chains were synthesized and inserted into the expression vector, followed by plasmid extraction. The plasmids were transiently transfected into ExpiCHO-S cells at a ratio of 1:1.

Cell Culture: ExpiCHO-S cells were thawed using TransMax C1 as the basal medium (manufactured by Mabion, catalog number MB1144.201), and cultured at 37°C with 8% $CO_2$. The cells were adjusted to $3\times10^6$ cells/mL one day before transfection, and further adjusted to $6\times10^6$ cells/mL on the day of transfection. The plasmid was added to OptiPRO™ SFM medium (manufactured by Gibco, catalog No: 12309-019) and mixed. Then FectoPRO transfection reagent (manufactured by Sartorius, catalog No: 101000007) was added and mixed. After incubation at room temperature for 10 min, the mixture was added dropwise to the cells.

The specific transfection system was as follows:

| Transfection volume (mL) | 500 |
| --- | --- |
| Sterile plasmid (light chain+ heavy chain) ($\mu$g) | 500 |
| OptiPRO™ SFM medium (mL) | 100 |
| FectoPRO transfection reagent ($\mu$L) | 3000 |

Feeding during culture process was as follows:

| Time | | D0 | D1 | D3 | D5 | D7 |
| --- | --- | --- | --- | --- | --- | --- |
| Culture process | | transfection | culturing at 32°C, 5% $CO_2$, and feeding | feeding | feeding | feeding |
| Feeding amount | Titer Maxer A (enhancer for target protein expression), calculated based on initial volume | / | 2% | / | / | / |
| | MaxFx feed medium, calculated based on initial volume | / | 3% | 3% | 3% | 3% |
| | MaxFB feed medium, calculated based on initial volume | / | 0.3% | 0.3% | 0.3% | 0.3% |
| | glucose, calculated based on volume after feeding | / | 5g/L | 5g/L | 5g/L | 5g/L |
| Note: Titer Maxer A (catalog No: MB7107.102), MaxFx (catalog No: MB1220.201), and MaxFB (catalog No: MB1202.201) were commercially available from Shanghai Mabion Biotechnology Co., Ltd. | | | | | | |

During the expression process, cell viability and density were detected, maintaining viability at no less than 60%. The expression supernatant was collected eight days post-transfection for subsequent purification.

Downstream purification process

In this example, protein Purification was performed using Protein A magnetic beads (commercially available from Nanjing GenScript Biotech Corporation, catalog No: L00695), including the following steps of:

- Bead preparation: 1 mL of magnetic bead ( pure bead volume of 25%) was pipetted into a 50 mL centrifuge tube, and washed three times with $1\times$ PBS for later use;
- Sample preparation: the expression supernatant was transferred into a 50 mL centrifuge tube, and centrifuged at 8,000 rpm for 30 min to remove impurities;
- Sample incubation: the centrifuged expression supernatant was transferred to pre-equilibrated magnetic beads, and incubated at room temperature for 1 to 2 hours to allow the antibody in the supernatant to fully bind to the magnetic beads;
- Washing: After the incubation is finished,, the 50 mL centrifuge tube was placed on a magnetic base to adsorb the magnetic beads, and wash it repeatedly with $1\times$PBS three times to remove impurities;
- Elution: 1 mL of 20 mM acetic acid was added into the centrifuge tube, mixed thoroughly, and incubated for 5 min at rest; then repeated twice to elute the target protein; and
- pH adjustion: the eluate was neutralized with 1 M Tris-HCl (pH 8.0) to obtain the final protein sample. The final purified protein was analyzed by SEC for purity. The remaining sample was stored at -20°C for later use.

**Example 2 Identification of mono-oxidized and di-oxidized variants in Apitegromab Sample**

The sample prepared in Example 1 was analyzed by liquid chromatography-tandem high-resolution mass spectrometry, including the following steps of:

- taking about 25µg of the sample, and adding urea at a final concentration of 6 M and TCEP at a final concentration of 10 mM, and incubateing at 37 °C for 20 min for denaturation and reduction;
- adding iodoacetamide at a final concentration of 20 mM and incubating at 37 °C for 15 min for alkylation to block free sulfhydryl groups ;
- adding 50 mM of ammonium bicarbonate solution to dilute the urea concentration to be 0.6 M, and then adding sequencing grade trypsin (available from Rhinogen, catalog No: QIP-003A) at a protein-to-enzyme ratio of 10:1 (w/w) for digestion at 37 °C for 25 min;
- after the enzymatic digestion is completed, adding formic acid at a final concentration of 1 % to terminate the digestion, and centrifuging the sample at 12000 rpm for 3 min; and transferring the supernate into sample vial;
- analyzing the test samples by liquid chromatography-tandem high-resolution mass spectrometry with an injection volume of 2 µg, and the conditions for chromatography and mass spectrometry are as follows.

Conditions for chromatography:

| LC system | UHPLC, Vanquish horizon, Thermo |
| --- | --- |
| Chromatography column | ACQUITY Premier BEH C18 column; 1.7µm partical size; 2.1×100mm |
| Column temperature | 60°C |
| Sample chamber temperature | 8°C |
| Mobile phase | phase A: a solution of 0.1% formic acid /water; phase B : a solution of 0.08% formic acid /80% acetonitrile / 20% water |
| Elution gradient | After 1% phase B was used for equilibration for 3 min, phase B increased linearly to 45% in 57 min |

Conditions for mass spectrometry:

| MS system | Orbitrap Exploris480, Thermo |
| --- | --- |
| Ionization mode | H-ESI positive |
| Capillary voltage | 3.5 kV |
| Ion transfer tube temperature | 300 °C |
| MS1 scan range | 200-2000 m/z |
| MS1 resolution | 90000 |
| MS1 AGC Target | 1e6 |
| MS2 resolution | 22500 |
| MS2 AGC Target | 1e5 |
| MS2 Isolation window | 1.6m/z |
| HCD collision energy (NCE) | 25% |

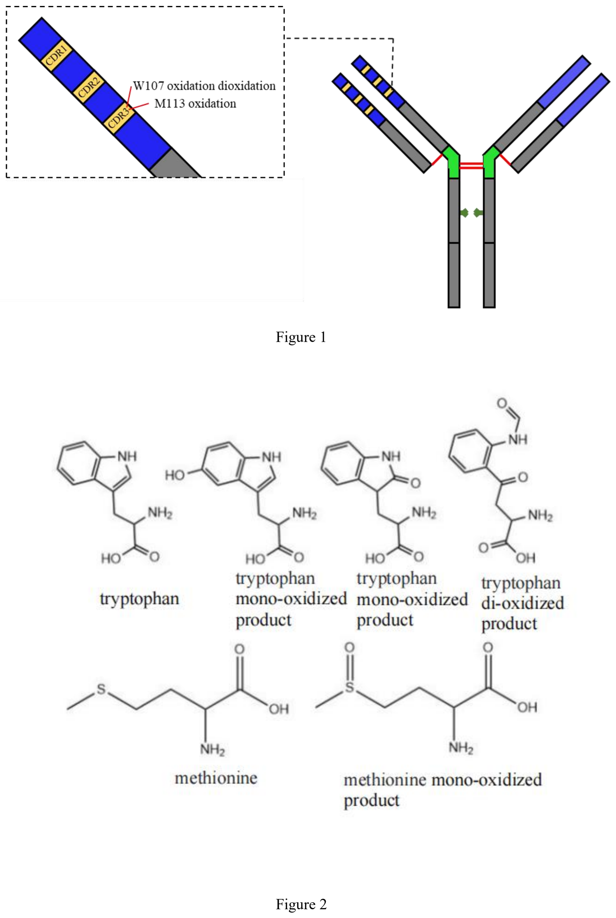

The structure of the oxidized variant of Apitegromab prepared in Example 1 was illustrated in FIG. 1, and the structures of tryptophan and methionine oxidized variants were illustrated in FIG. 2, including the tryptophan mono-oxidized variant, the tryptophan di-oxidized variant, and the methionine mono-oxidized variant. Among them, the molecular weight of the mono-oxidized variant increased by 16 Da, and the molecular weight of the di-oxidized variant increased by 32 Da.

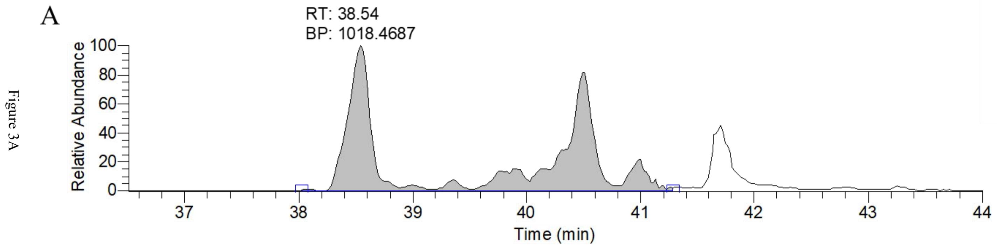

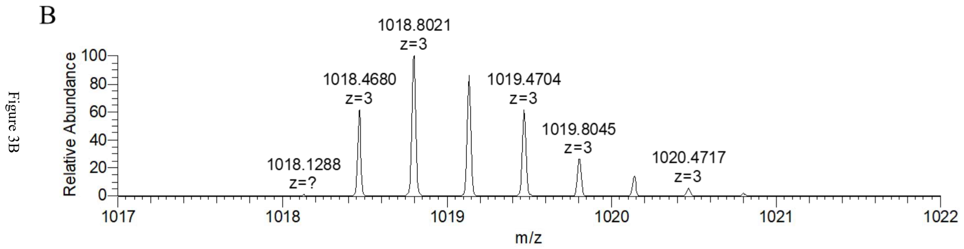

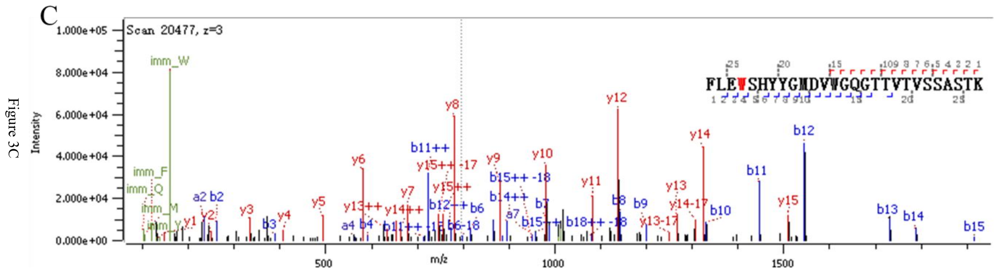

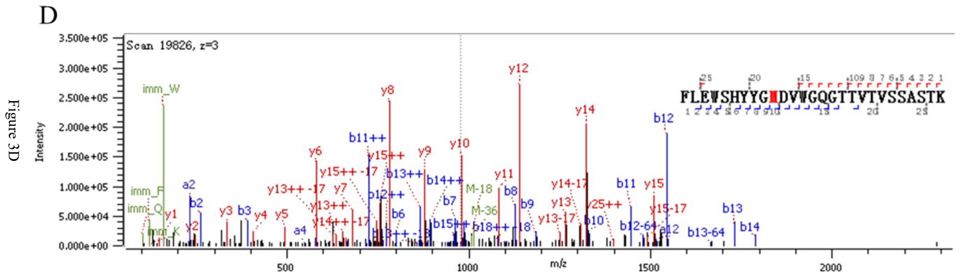

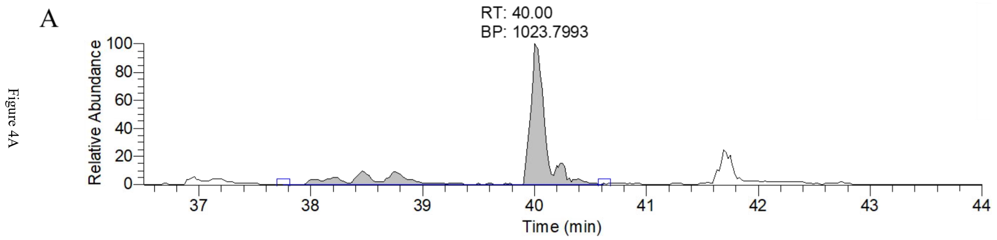

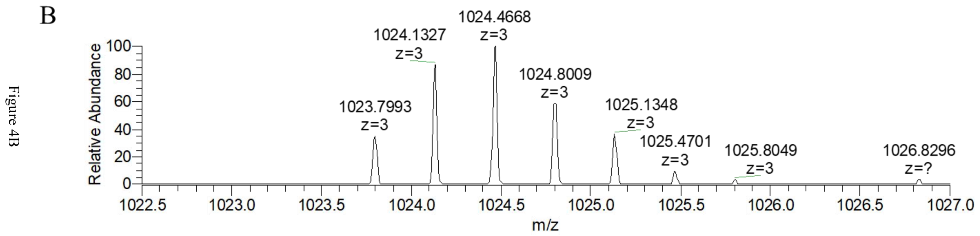

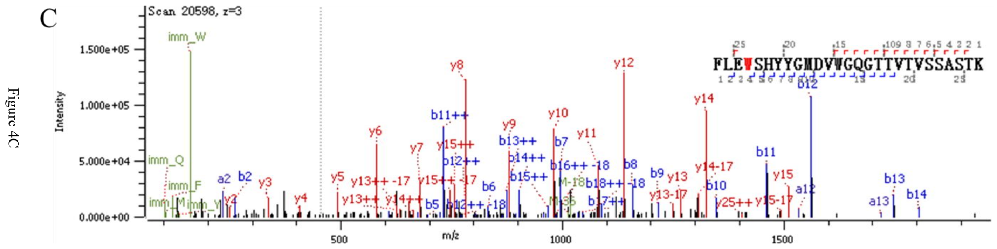

The modification sites were identified using Byos software (Protein Metrics), and extracted ion chromatograms were obtained using Qual Browser of Xcalibur software. Extracted ion chromatogram (XIC) of MS1, MS1 spectrum, and MS2 spectrum of W107 and M113 mono-oxidized variants were illustrated in FIG. 3, and extracted ion chromatogram (XIC) of MS1, MS1 spectrum, and MS2 spectrum of the W107 di-oxidized variant were illustrated in FIG. 4.

The above analysis results showed that an oxidized form of the peptide segment FLEWSHYYGMDVWGQGTTVTVSSASTK (SEQ ID NO: 4) which contains part of the CDR3 sequence of the heavy chain was present in the sample (underlined portions present modification sites: W and M have mono-oxidized forms, and W has di-oxidized form). For the mono-oxidized peptide segment, the measured single-isotope peak $m/z$ is 1018.4680, and the theoretical $m/z$ is 1018.4710, with an error of only -2.95ppm. For the di-oxidized peptide segment, the measured single-isotope peak $m/z$ of is 1023.7993, and the theoretical m/z is 1023.8027, with an error of only -3.32ppm. Additionally, the fragment ions in the MS2 spectrum match well with the theoretical b and y ions of the modified peptide segment, confirming the modification sites and forms of the peptide segment.

**Example 3 Contents of mono-oxidized and di-oxidized variants in Apitegromab Sample**

Peptide mass fingerprinting analysis was performed on samples prepared in Example 1 by LC-MS/MS. After the mass spectrometry data was obtained, XIC chromatograms of the mono-oxidized modification form (the mass-to-charge ratios of the main ions are 1018.47 and 1527.20, respectively), di-oxidized modification form (the mass-to-charge ratios of the main ions are 1023.80 and 1535.20, respectively), and its corresponding unmodified form (also named as "native peptide segment", the mass-to-charge ratios of the main ions are 1013.14 and 1519.21, respectively) of the peptide segment FLEWSHYYGMDVWGQGTTVTVSSASTK (SEQ ID NO: 4) are extracted respectively, and integrated to obtain respective peak areas of each target component. The contents of the mono-oxidized variant and di-oxidized variant were calculated according to the following formulas:

The content of mono-oxidized variant = (XIC area of mono-oxidized peptide segment)/(XIC area of mono-oxidized peptide segment + XIC area of di-oxidized peptide segment + XIC area of native peptide segment) × 100% The content of di-oxidized variant = (XIC area of di-oxidized peptide segment)/(XIC area of mono-oxidized peptide segment + XIC area of di-oxidized peptide segment + XIC area of native peptide segment) × 100%

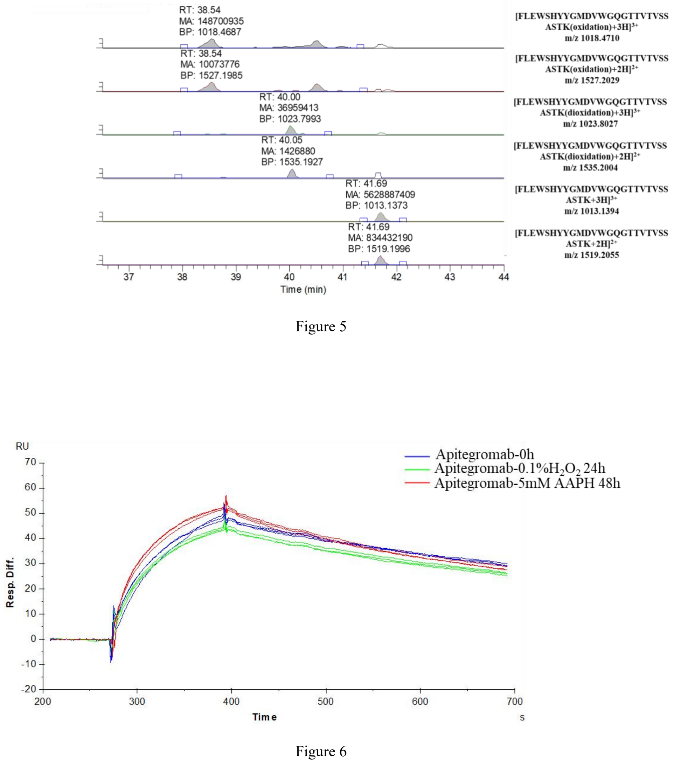

The method for sample pre-processing and the method for LC-MS/MS detection were as described in Example 2. Data processing was performed using Qual Browser (Xcalibur software). The XIC chromatograms of the mono-oxidized peptide segment, the di-oxidized peptide segment, and the native peptide segment were illustrated in FIG. 5.

The contents of the mono-oxidized and di-oxidized variants in the sample were calculated according to the following formulas, and results were shown in Table 1.

The content of mono-oxidized variant = [(148700935 + 10073776)/(148700935 + 10073776 + 36959413 + 1426880 + 5628887409 + 8 34432190)] × 100% The content of di-oxidized variant = [(36959413 + 1426880)/(148700935 + 10073776 + 36959413 + 1426880 + 5628887409 + 834 432190)] × 100%

Table 1. Contents of mono-oxidized and di-oxidized variants in Apitegromab sample

| Variant form | Variant content (%) |
| --- | --- |
| HC:W[107]/M[113] mono-oxidation | 2.38 |
| HC:W[107] di-oxidation | 0.58 |
| Note: "/" represents that the variant content is the sum of the HC:W[107] mono-oxidized variant and the HC: M[113] mono-oxidized variant. | |

**Example 4 Preparation of Apitegromab forced oxidation sample, and identification of the contents of mono-oxidized and di-oxidized variants**

The samples prepared in Example 1 were subjected to forced oxidation using different oxidants respectively to obtain forced oxidation samples with higher contents of mono-oxidized and di-oxidized variants.

Forced oxidation with AAPH: The protein sample was added with 2,2'-azobis(2-amidinopropane) dihydrochloride (AAPH)at a final concentration of 5 mM , and incubated at room temperature for 48 hours to perform forced oxidation. After the forced oxidation reaction is completed, the protein sample was buffer-exchanged into PBS using a 10 kDa ultrafiltration tube to remove AAPH from the system.

Forced oxidation with $H_2O_2$: The protein sample was added with hydrogen peroxide ($H_2O_2$)at a final concentration of 0.1%, and incubated at room temperature for 24 hours to perform forced oxidation. After the forced oxidation reaction is completed, the protein sample was buffer-exchanged into PBS using a 10 kDa ultrafiltration tube to remove $H_2O_2$ from the system.

Peptide mass fingerprinting analysis was performed on the forced oxidation samples prepared by LC-MS/MS. The method for sample pre-processing and the method for LC-MS/MS detection were as described in Example 2. Data processing was as described in Example 3. Results were shown in Table 2.

Table 2. Contents of mono-oxidized and di-oxidized variants in forced oxidation Apitegromab sample

| Variant form | Variant content (%) | |
|---|---|---|
| | 5mM AAPH, 48h | 0.1% $H_2O_2$, 24h |
| HC:W[107]/M[113] mono-oxidation | 32.41 | 38.20 |
| HC:W[107] di-oxidation | 41.58 | 0.68 |
| Note: "/" represents that the variant content is the sum of the HC:W[107] mono-oxidized variant and the HC: M[113] mono-oxidized variant. | | |

The results showed that treatment of an Apitegromab sample with AAPH significantly increased the contents of both mono-oxidized and di-oxidized variants, and HC:W[107] mono-oxidation was the main component in the mono-oxidized varaints; and treatment of an Apitegromab sample with $H_2O_2$ significantly increased the content of the mono-oxidized variant, and HC:M[113] mono-oxidation was the main component in the mono-oxidized varaints.

**Example 5 Effect of oxidized variant on Apitegromab**

A comparative study on antigen-binding affinity of Apitegromab samples prepared in Example 1 and Example 4 was performed. The SPR technique was used to determine the affinity of the original samples and the forced oxidation samples for the antigen Myostatin. The specific steps are as follows.

Analysis was carried out using the His capture method. His-tagged Myostatin was immobilized on the reference and experimental channels of a CM5 chip via amino coupling, with a capture amount of about 74 RU. Next, 50 nM diluted Apitegromab was sequentially flowed through the reference and experimental channels for binding (120 seconds) and dissociation (300 seconds). After each cycle, the chip was regenerated with 50 mM hydrochloric acid. The resulting sensorgrams were analyzed by fitting a 1:1 binding model using Biacore™ Insight Evaluation software. Each sample was subjected to three parallel dilutions and affinity measurements.

To investigate whether the oxidized variants affected antibody-antigen binding, the KD values for antigen binding affinity were simultaneously measured for two types of forced-oxidation samples and untreated samples. The results are presented in Table 3, and the sensorgrams are shown in Figure 6. The KD value range of the affinity of the untreated samples for recombinant human Myostatin is 2.75-4.00 nM. The KD value range of the affinity of the samples subjected to $H_2O_2$ forced oxidation for recombinant human Myostatin is 3.74 - 4.08 nM. The KD value range of the affinity of the samples subjected to AAPH forced oxidation for recombinant human Myostatin is 3.37 - 4.13 nM. These values are basically consistent, indicating that the antibody-antigen affinity did not change significantly before and after the forced oxidation reaction.

Table 3 Antigen-binding affinity of Apitegromab comprising different proportions of oxidized variants

| Sample information | ka (1/Ms) | kd (1/s) | $K_D$ (M) |
|---|---|---|---|
| Apitegromab-0h-1 | 9.37E+05 | 0.002575 | 2.75E-09 |
| Apitegromab-0h-2 | 3.77E+05 | 0.001506 | 4.00E-09 |

(continued)

| Sample information | ka (1/Ms) | kd (1/s) | $K_D$ (M) |
|---|---|---|---|
| Apitegromab-0h-3 | 4.17E+05 | 0.00134 | 3.21E-09 |
| Apitegromab-0.1%$H_2O_2$ 24h-1 | 3.97E+05 | 0.001549 | 3.90E-09 |
| Apitegromab-0.1%$H_2O_2$ 24h-2 | 4.26E+05 | 0.001592 | 3.74E-09 |
| Apitegromab-0.1%$H_2O_2$ 24h-3 | 4.05E+05 | 0.001654 | 4.08E-09 |
| Apitegromab-5mM AAPH 48h-1 | 4.59E+05 | 0.001899 | 4.13E-09 |
| Apitegromab-5mM AAPH 48h -2 | 5.41E+05 | 0.001824 | 3.37E-09 |
| Apitegromab-5mM AAPH 48h -3 | 4.94E+05 | 0.001964 | 3.98E-09 |

It is generally believed that post-translational modifications in the CDR3 region of an antibody can potentially affect antigen binding properties by altering the microenvironment of the binding interface. This study evaluated the effect of site-specific oxidation modifications (including mono-oxidized and di-oxidized variants) at positions W107 and M113 in the CDR3 region of the heavy chain of Apitegromab on antigen binding. The results showed that even when the content of these oxidized variants was elevated to higher levels under forced oxidation conditions, their antigen-binding affinity showed no significant difference from that of the unmodified form. Based on this finding, there is no need to implement a targeted removal strategy for the oxidized variant impurities at positions W107 and M113 during the process development of Apitegromab.

Various changes and equivalent substitutions can be made to the embodiments disclosed herein without departing from the spirit and scope of the disclosure. Unless otherwise indicated in the context, any feature, step, or embodiment of the disclosed embodiments can be used in combination with any other features or embodiments.

**Claims**

1. A composition comprising Apitegromab and a variant thereof, wherein the variant comprises a mono-oxidized variant resulting from oxidation of tryptophan at position 107 in the amino acid sequence of the heavy chain of Apitegromab, and a mono-oxidized variant resulting from oxidation of methionine at position 113 in the amino acid sequence of the heavy chain of Apitegromab, and
   wherein the positions are numbered with reference to the sequence as set forth in SEQ ID NO:1.

2. A composition comprising Apitegromab and a variant thereof, wherein the variant comprises a di-oxidized variant resulting from oxidation of tryptophan at position 107 in the amino acid sequence of the heavy chain of Apitegromab, and
   wherein the position is numbered with reference to the sequence as set forth in SEQ ID NO:1.

3. The composition of claim 1, wherein the variant is a mono-oxidized variant resulting from oxidation of tryptophan at position 107 in the amino acid sequence of the heavy chain of Apitegromab, and a mono-oxidized variant resulting from oxidation of methionine at position 113 in the amino acid sequence of the heavy chain of Apitegromab.

4. The composition of claim 2, wherein the variant is a di-oxidized variant resulting from oxidation of tryptophan at position 107 in the amino acid sequence of the heavy chain of Apitegromab.

5. The composition of claim 1, wherein the variant further comprises a di-oxidized variant resulting from oxidation of tryptophan at position 107 in the amino acid sequence of the heavy chain of Apitegromab.

6. The composition of claim 1, wherein the sum of the contents of the two mono-oxidized variants is less than or equal to about 40% of the total content of Apitegromab and variants thereof in the composition, calculated based on a peptide mass fingerprinting analysis.

7. The composition of claim 2, wherein the content of the di-oxidized variant is less than or equal to about 45% of the total content of Apitegromab and variants thereof in the composition, calculated based on a peptide mass fingerprinting analysis.

8. The composition of any one of claims 1 to 7, wherein the variant has essentially similar antigen-binding affinity to Apitegromab.

9. A pharmaceutical formulation, comprising the composition of any one of claims 1 to 7, and one or more pharmaceutically acceptable carriers.

10. A method for detecting an Apitegromab variant in a composition or a pharmaceutical formulation comprising Apitegromab, wherein the variant is i) a mono-oxidized variant resulting from oxidation of tryptophan at position 107 in the amino acid sequence of the heavy chain of Apitegromab, and a mono-oxidized variant resulting from oxidation of methionine at position 113 in the amino acid sequence of the heavy chain of Apitegromab; or ii) a di-oxidized variant resulting from oxidation of tryptophan at position 107 in the amino acid sequence of the heavy chain of Apitegromab, and wherein the positions are numbered with reference to the sequence as set forth in SEQ ID NO:1; and wherein the method comprises:

subjecting the composition or pharmaceutical formulation to trypsin digestion to obtain the digestion product; subjecting the digestion product to a peptide mass fingerprinting analysis, for example, by using LC-MS/MS, and determining the presence or absence of the variant based on MS1 and MS2 spectra.

11. The composition of any one of claims 1 to 7 for use in treating a neuromuscular disease.

12. The composition of claim 11, wherein the neuromuscular disease is a muscular atrophy disease.

13. The composition of claim 11, wherein the neuromuscular disease is spinal muscular atrophy, sarcopenia or Duchenne muscular dystrophy.

Figure 1

Figure 2

Figure 3A

Figure 3B

Figure 3C

EP 4 763 222 A1

Figure 3D

Figure 4A

Figure 4B

Figure 4C

EP 4 763 222 A1

Figure 5

Figure 6

Europäisches
Patentamt

European
Patent Office

Office européen
des brevets

# EUROPEAN SEARCH REPORT

Application Number

EP 25 21 1886

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2022/093724 A1 (SCHOLAR ROCK INC [US]) 5 May 2022 (2022-05-05) * claim 1 * ----- | 1-9, 11-13 | INV. A61K39/395 C07K16/22 |
| X | FISCHER PAULINA ET AL: "Antibody oxidation and impact of formulation: A high-throughput screening approach", EUROPEAN JOURNAL OF PHARMACEUTICAL SCIENCES- AUTHOR MANUSCRIPT, vol. 209, 1 June 2025 (2025-06-01), page 107113, XP093392640, NL ISSN: 0928-0987, DOI: 10.1016/j.ejps.2025.107113 * abstract * ----- | 10 | |

TECHNICAL FIELDS
SEARCHED (IPC)

A61K
C12Q
C07K
G01N

The present search report has been drawn up for all claims

| Place of search | Date of completion of the search | Examiner |
|---|---|---|
| The Hague | 30 April 2026 | Patti, Gabriele |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 21 1886

This annex lists the patent family members relating to the patent documents cited in the above-mentioned European search report.
The members are as contained in the European Patent Office EDP file on
The European Patent Office is in no way liable for these particulars which are merely given for the purpose of information.

30-04-2026

| Patent document cited in search report | Publication date | Patent family member(s) | | Publication date |
|---|---|---|---|---|
| WO 2022093724 A1 | 05-05-2022 | AU | 2021369471 A1 | 15-06-2023 |
| | | CA | 3193909 A1 | 05-05-2022 |
| | | CN | 116390756 A | 04-07-2023 |
| | | EP | 4232151 A1 | 30-08-2023 |
| | | EP | 4729069 A1 | 22-04-2026 |
| | | ES | 3058087 T3 | 06-03-2026 |
| | | IL | 302132 A | 01-06-2023 |
| | | JP | 2023549455 A | 27-11-2023 |
| | | KR | 20230095998 A | 29-06-2023 |
| | | PL | 4232151 T3 | 23-02-2026 |
| | | TW | 202233234 A | 01-09-2022 |
| | | US | 2024002490 A1 | 04-01-2024 |
| | | WO | 2022093724 A1 | 05-05-2022 |

EPO FORM P0459

For more details about this annex : see Official Journal of the European Patent Office, No. 12/82

**EP 4 763 222 A1**

REFERENCES CITED IN THE DESCRIPTION

**Patent documents cited in the description**

- CN 202511190218